(19) Europäisches Patentamt
European Patent Office
Office européen des brevets

(11) EP 3 046 903 B1

(12) **EUROPÄISCHE PATENTSCHRIFT**

(45) Veröffentlichungstag und Bekanntmachung des Hinweises auf die Patenterteilung:
**24.03.2021 Patentblatt 2021/12**

(21) Anmeldenummer: **14771817.5**

(22) Anmeldetag: **10.09.2014**

(51) Int Cl.:
***C07C 319/20*** *(2006.01)* ***C07C 319/26*** *(2006.01)*
***C07C 319/28*** *(2006.01)* ***C07C 323/58*** *(2006.01)*

(86) Internationale Anmeldenummer:
**PCT/EP2014/069247**

(87) Internationale Veröffentlichungsnummer:
**WO 2015/039935 (26.03.2015 Gazette 2015/12)**

(54) **VERFAHREN ZUR GEWINNUNG VON METHIONIN**

METHOD FOR EXTRACTING METHIONINE

PROCÉDÉ DE COLLECTE DE MÉTHIONINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.09.2013 EP 13184831**

(43) Veröffentlichungstag der Anmeldung:
**27.07.2016 Patentblatt 2016/30**

(73) Patentinhaber: **Evonik Operations GmbH**
**45128 Essen (DE)**

(72) Erfinder:
- **REICHERT, Stefan**
**60438 Frankfurt (DE)**
- **JAKOB, Harald**
**63594 Hasselroth (DE)**
- **HASSELBACH, Hans Joachim**
**63571 Gelnhausen (DE)**
- **KÖRFER, Martin**
**63796 Kahl (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**WO-A1-03/050071 JP-A- 11 158 140**
**US-A1- 2005 176 115**

- **"Dl-methionine crystallization", WPI / THOMSON,, Bd. 1971, Nr. 21, 1. Januar 1971 (1971-01-01), XP002681651, & JP 46- 019 610 B 1971**
- **CANSELIER J P: "THE EFFECTS OF SURFACTANTS ON CRYSTALLIZATION PHENOMENA", JOURNAL OF DISPERSION SCIENCE AND TECHNOLOGY, TAYLOR AND FRANCIS GROUP, NEW YORK, NY, US, Bd. 14, Nr. 6, 1. Januar 1993 (1993-01-01) , Seiten 625-644, XP008011857, ISSN: 0193-2691, DOI: 10.1080/01932699308943435**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 046 903 B1

## Beschreibung

Gegenstand der Erfindung ist ein Verfahren zur Isolierung von D,L-Methionin mit Schüttgewichten > 550 g/l aus D,L-Methioninammoniumsalz -haltigen Lösungen.

## Stand der Technik

Verfahren zur Herstellung von D, L-Methionin, bei denen das D,L-Methionin zunächst als Ammoniummethioninat anfällt, sind bekannt.

Nach US20050176115 wird eine wässrige, ammoniumhaltige D,L-Methioninlösung erhalten, indem wässrige Lösungen von 2-Amino-4-methylthiobutyronitril bereits in Gegenwart von $NH_3$ mit Biokatalysatoren zum D,L-Methionin umgesetzt werden. Das D,L-Methionin wird anschließend durch Abtrennen des Ammoniaks bei verringertem Druck ausgefällt. Das so erhaltene D,L-Methionin weist eine Reinheit von 99% auf. Das Schüttgewicht des erhaltenen D,L-Methionins wird nicht angegeben.

In JP2004-254690 wird ebenfalls durch Umsetzung von 2-Amino-4-methylthiobutyronitril mit einem Biokatalysator D,L-Methionin und $NH_3$ gebildet. Durch die Gegenwart des Ammoniaks wird die Löslichkeit des Methionins erhöht, was die Abtrennung des Biokatalysators erleichtert. Über die genauen Isolierungsbedingungen sowie die Produkteigenschaften des D,L-Methionins wird in JP2004-254690 keine Aussage gemacht.

DE 60127538 beschreibt ein Verfahren bei dem D,L-Methionin durch eine katalysierte Verseifungsreaktion aus Methioninamid erzeugt wird. Aus der entstandenen Ammoniummethioninatlösung wird hier der Ammoniak durch Strippen vollständig entfernt und das Methionin auskristallisiert, wobei keine Schüttgewichte des erhaltenen Methionins angegeben werden.

In WO 2008006977 wird D,L-Methionin über eine Verseifung mit $NH_3$ aus dem Methioninhydantoin gewonnen und durch Abdampfen von $NH_3$ und $CO_2$ bei vermindertem Druck wird das D,L-Methionin gewonnen. Über die Produkteigenschaften wird nichts gesagt.

WO2007034065 beschreibt ebenfalls eine ammoniakalische Verseifung von Methioninhydantoin. Aus der Ammoniummethioninatlösung wird der Ammoniak in einer Strippkolonne entfernt und anschließend das Methionin durch abkühlen der Lösung ausgefällt. Es findet sich keine Aussage zu den Produkteigenschaften des gebildeten Methionins.

DE 10238212 beschreibt ein Verfahren bei dem Methioninhydantoin in Wasser mit oder ohne Katalysator bei hohen Temperaturen verseift wird. Vor der Kristallisation des entstandenen Methionins wird $NH_3$ und $CO_2$ teilweise abgetrennt. Es werden weder die Restmengen an $CO_2$ und Ammoniak in der Lösung aus der die Kristallisation erfolgt genannt, noch wird etwas über das Schüttgewicht des erhaltenen Methionins ausgesagt.

Die WO 2003050071 beschreibt wässrige Mischungen von Fettsäurepolyethylenglycolester mit modifizierten Cellulosen, die als Hilfsmittel bei der Kristallisation von Methionin aus mit Kohlendioxid neutralisierten Kaliummethioninatlösungen aus der alkalischen Methionin-Hydantoin-Verseifung verwendet werden. Bei diesem speziellen Verfahren in Gegenwart großer Kaliummengen werden Schüttgewichte von bis zu 586g/l, in einem Fall von 620g/l, erhalten (mit Additiv Hydroxyethylcellulose). Es werden jedoch keine Angaben gemacht zur Kristallisation von D,L-Methionin aus D,L-Ammoniummethioninat enthaltenden Lösungen.

Die Löslichkeit von D,L-Methionin wird in Gegenwart von $NH_3$ erhöht (siehe Löslichkeitskurven aus JP2004-254690).

Für ein wirtschaftliches Verfahren zur Isolierung von D, L-Methionin ist es demnach zweckmässig, den Ammoniak möglichst vollständig zu entfernen, um die Menge an kristallisierbarem D,L-Methionin zu maximieren. Hierzu kann der Ammoniak durch verschiedene literaturbekannte Verfahren wie Strippen, Abdampfen bei vermindertem Druck etc. entfernt werden.

## Aufgabe der Erfindung

Die Aufgabe der Erfindung ist es eine Methode zur Isolierung von D, L-Methionin ausgehend von Ammoniummethioninat enthaltenden Lösungen bereitzustellen, bei der das D,L-Methionin mit hohen Schüttgewichten > 550 g/l erhalten wird.

## Beschreibung

Bei den vorliegenden Untersuchungen hat sich gezeigt, dass das D,L-Methionin bei einer Kristallisation aus wässrigen Lösungen üblicherweise mit niedrigen Schüttgewichten < 550 g/l erhalten wird. Durch die alleinige Zugabe von Kristallisationsadditiven konnte keine signifikante Erhöhung des Schüttgewichtes erreicht werden. Überraschenderweise hat sich gezeigt, dass eine Restmenge $NH_3$ in Kombination mit zugesetzten Additiven zu D,L-Methionin mit höherem Schüttgewicht führt.

Da sich in wässrigen Lösungen, die sowohl $NH_3$ als auch Methionin enthalten ein Gleichgewicht zwischen Ammoniummethioninat einerseits und Methionin und Ammoniak andererseits einstellt, wird zur Vereinfachung im Folgenden nur noch über $NH_4^+$-Konzentrationen gesprochen, unabhängig davon, ob es sich dabei um $NH_4^+$ oder $NH_3$ handelt.

Die oben genannte Aufgabe sowie weitere damit im Zusammenhang stehende aber nicht explizit genannte Aufgaben werden gelöst, durch Bereitstellung eines Verfahrens zur Kristallisation von D,L-Methionin aus D,L-Methionin und D,L-Methioninammoniumsalz enthaltenden wässrigen Lösungen und/oder Suspensionen mit einem Met-Gehalt (umfassend das gesamte Methionin in Form von D,L-Methionin und D,L-Methioninammoniumsalz) von 70 - 180 g/kg der Lösung und/oder Suspension (7 - 18 Gew.-%), vorzugweise 90 - 150 g/kg (9 - 15 Gew.-%), einem $NH_4^+$-Gehalt von 1 - 5 g/kg der Lösung und/oder Suspension (0,1 - 0,5 Gew.-%), vorzugsweise 1,5 bis 3,0 g/kg (0,15 - 0,3 Gew.%), in Gegenwart eines Kristallisationsadditivs,
welches ein anionisches Tensid oder eine Mischung aus verschiedenen anionischen Tensiden umfasst,
bei dem die Temperatur der Lösung und/oder Suspension von einem Temperaturbereich $T_1$ = 85 - 110°C direkt oder schrittweise auf einen Temperaturbereich $T_2$ = 30 - 50°C abgesenkt wird, so dass Methionin als Feststoff aus der Lösung und/oder Suspension ausfällt.

Unter D,L-Methionin und D,L-Methioninammoniumsalz enthaltender wässriger Lösung wird hier verstanden, dass der überwiegende Anteil des gesamten Methionins (Met) gelöst vorliegt und nur geringe Met-anteile von max. 5% ungelöst, also suspendiert vorliegen.

Unter D,L-Methionin und D,L-Methioninammoniumsalz enthaltender wässriger Suspension wird hier verstanden, dass ein deutlicher Anteil nämlich von > 5% des gesamten Methionins suspendiert vorliegt, während der Rest in gelöster Form vorliegt.

Entsprechend liegt die optimale $NH_4^+$-Konzentration bezogen auf Methionin bei mindestens ca. 5 g $NH_4^+$ / kg Met und bei maximal ca. 60 g $NH_4^+$ / kg Met wenn die Methioninkonzentration in der Lösung im Bereich von 90 g/kg bis 150 g/kg liegt.

Die $NH_4^+$-Konzentration kann dabei beispielsweise mit einer $NH_4^+$-sensitiven Elektrode nach bekannten Verfahren bestimmt werden. Die $NH_4^+$-Konzentration wird hier in der Regel durch Messung an einer auf pH 11 eingestellten Probenlösung und Vergleich mit der Messung an ebenfalls auf pH 11 eingestellten $NH_4Cl$-Lösungen bekannter Konzentration ermittelt.

Die Methioninkonzentration in der Lösung und/oder Suspension wird am einfachsten per HPLC bestimmt.

Durch die beschriebene Kombination aus der Gegenwart von $NH_4^+$-Ionen, der Zugabe von Kristallisationsadditiv und der Temperatursteuerung der Kristallisation werden mit dem erfindungsgemäßen Verfahren grobkörnige, gut filtrierbare Methioninkristalle mit einem Schüttgewicht von > 550 g/l nach Trocknung erhalten.

Als anionische Tenside eignen sich insbesondere Tenside nach einer der in Formel 1 bis 3 dargestellten Verbindungen, oder Mischungen daraus:

$R^1$-O-$SO_3M$ (Formel 1)

$R^2$-O-$(CH_2)_n$-$SO_3M$ (Formel 2)

$R^3$-$(O\text{-}C_2H_4)_n$-O-$SO_3M$ (Formel 3)

wobei n eine ganze Zahl von 1 bis 10, M Natrium oder Kalium und $R^1$, $R^2$ und $R^3$ eine lineare, verzweigte oder cyclische, gesättigte oder ungesättigte $C_8$- bis $C_{20}$-Alkylgruppe oder eine Arylgruppe ist. Mit diesen wurden, wie die Beispiel 1 mit den Zusätzen 2, 3 und 4 in Tabelle 1 zeigt, relativ hohe Schüttgewichte von 564 bis 588 kg/l erhalten.

Vorzugsweise verwendet werden Tenside, bei denen n gleich 2 und $R^1$, $R^2$ und $R^3$ lineare, gesättigte $C_8$- bis $C_{18}$- Alkylgruppen sind, weil diese kommerziell leicht erhältlich und wirksam sind.
Besonders bevorzugt eingesetzt werden anionische Tenside, der Formeln $C_nH_{2n+1}$-O-$SO_3Na$, mit n = 12 bis 18 (Sulfopon® 1218G, Oleochemicals) $C_nH_{2n+1}$-O-$C_2H_4$-$SO_3Na$, mit n = 8 bis 18 (Hostapon® SCI 85, Clariant) $C_nH_{2n+1}$-$(OC_2H_4)_2$-O-$SO_3Na$, mit n = 12 (Disponil® FES 27, Cognis)

Die Konzentration des Kristallisationsadditivs (auf Basis des Wirkstoffs) in der Lösung und/oder Suspension, aus der die Kristallisation erfolgt, beträgt vorzugsweise mindestens 700 ppm und maximal 4000 ppm bezogen auf die Gesamtmasse der Lösung und/oder Suspension, besonders bevorzugt mindestens 750 ppm und maximal 2000 ppm, am meisten besonders bevorzugt mindestens 800 ppm und maximal 1000 ppm. Dies gewährleistet eine gute Entfaltung der Wirkung des Additivs ohne zu viel an Fremdstoffen in die Produktlösung einzutragen.
Um eine optimale Dosierung und Verteilung des Kristallisationsadditivs zu erreichen, wird dieses vorzugsweise in Form einer wässrigen Lösung oder Emulsion eingesetzt, wobei die Konzentration des Kristallisationsadditivs in der Lösung oder Emulsion bevorzugt 2 bis 15 Gewichts-% beträgt.

In einer bevorzugten Ausführungsform des erfindungsgemäßen Verfahrens enthält die Lösung, aus der die

Kristallisation erfolgt, zusätzlich einen Entschäumer. Der Entschäumer hat die Funktion den bei der Handhabung der Methioninlösung und/oder -suspension entstehenden und von einigen der oben genannten Kristallisationsadditive verstärkten bzw. verursachten Schaum niederzuschlagen. Darüber hinaus ergibt sich bei einem gleichzeitigen Einsatz von Entschäumer und Kristallisationsadditiven überraschenderweise ein synergistischer Effekt bei den erzielten Schüttgewichten des Methionins, wodurch Schüttgewichte von sogar über 600 g/l erreicht und gleichzeitig die negativen Auswirkungen von Anreicherungsprozessen vermieden werden und damit das erfindungsgemäße Verfahren auch in kontinuierlicher Verfahrensweise durchführbar ist. Dies zeigt sich insbesondere durch Vergleich der Versuche 2, 3 bzw. 4 (ohne zusätzlichen Entschäumer -> Schüttgewichte von 564-588 kg/l) ) mit den Versuchen 8, 9 bzw. 10 (mit zusätzlichem Entschäumer -> Schüttgewichte von 630- 634 kg/l) in Tabelle 1 von Beispiel 1. Zusätzlich ermöglicht diese Ausführungsform weniger Kristallisationsadditiv einzusetzen.

Vorzugsweise eingesetzt werden Entschäumer, die Silikonöl enthalten, da diese sich als besonders effektiv erwiesen haben, wobei bevorzugt ein Silikonöl mit einer kinematischen Viskosität von 0,65 bis 10000 $mm^2/s$ (gemessen bei 25°C gemäß DIN 53018), besonders bevorzugt von 90 bis 1500 $mm^2/s$ eingesetzt wird. Der Entschäumer kann weiterhin Bestandteile enthalten, die als Emulgatoren wirksam sind, beispielsweise Mischungen polyethoxylierter Fettsäuren und polyethoxylierter Fettalkohole. Der Entschäumer kann ebenfalls Kieselsäure enthalten. In einer bevorzugten Ausführungsform ist der Entschäumer eine wässrige Lösung, die 5 bis 10 Gewichts-% Silikonöl, 0,05 bis 1 Gewichts-% Kieselsäure, 0,5 bis 5 Gewichts-% einer Mischung polyethoxylierter Fettsäuren, sowie 2 bis 7 Gewichts-% einer Mischung polyethoxylierter Fettalkohole enthält.

Vorzugsweise wird der Entschäumer in einer Mischung mit dem Kristallisationsadditiv eingesetzt. Um eine kontinuierliche, stabile Dosierung des Entschäumers zu erreichen, wird er vorzugsweise vor seinem Einsatz noch weiter mit Wasser verdünnt.

Die Verwendung von Silikonölentschäumern führt dazu, dass in dem nach dem erfindungsgemäßen Verfahren hergestellten Methionin mit einem geeigneten Analysenverfahren (z. B. Röntgenphotoelektronenspektroskopie, kurz XPS) Silicium nachgewiesen werden kann.

Bevorzugt verwendet wird der Entschäumer im erfindungsgemäßen Verfahren dabei so, dass das Gewichtsverhältnis des Entschäumers: Kristallisationsadditiv (auf Basis des Wirkstoffs) in der Lösung bzw. Suspension, aus der die Kristallisation erfolgt, im Bereich von 4:1 bis 1:1, vorzugsweise im Bereich von 3:1 bis 2:1 liegt und dabei die Konzentration des Kristallisationsadditivs (auf Basis des Wirkstoffs) mindestens 50 ppm und maximal 1200 ppm bezogen auf die Gesamtmasse der Lösung und/oder Suspension beträgt, vorzugsweise 100 ppm bis 600 ppm, besonders bevorzugt 200 ppm bis 400 ppm. Die dabei erzielten hohen D,L-Methionin-Schüttgewichte von deutlich über 600 g/l lassen sich besonders gut in Beispiel 4/Tabelle 3 erkennen.

Das erfindungsgemäße Verfahren wird dabei vorzugsweise so geführt, dass die Kristallisation durch Einleiten einer 85 bis 110°C heißen D,L-Methionin und D,L-Methioninammoniumsalz enthaltenden wässrigen Lösung und/oder Suspension in eine 30 bis 50°C warme D,L-Methionin und D,L-Methioninammoniumsalz enthaltenden wässrigen Lösung und/oder Suspension erfolgt, wobei die Temperatur der dabei entstehenden Mischung konstant zwischen 30 und 50°C gehalten wird.

Ganz besonders bevorzugt ist es dabei, eine 85 bis 110°C heiße D,L-Methionin- und D,L-Methioninammoniumsalz enthaltende wässrige Lösung in eine 30 bis 50°C warme D,L-Methionin und D,L-Methioninammoniumsalz enthaltende wässrige Suspension einzuleiten. Dies hat den Vorteil, dass besonders gut filtrierbare Kristalle erhalten werden.

Eine weitere bevorzugte Ausführungsform des Verfahrens ist dadurch gekennzeichnet, dass die Kristallisation zweistufig durchgeführt wird, wobei in der ersten Kristallisationsstufe eine 85 bis 110°C heiße D,L-Methionin und D,L-Methioninammoniumsalz enthaltende Lösung und/oder Suspension in eine 60 bis 80 °C warme D,L-Methionin und D,L-Methioninammoniumsalz enthaltende Suspension eingeleitet und die Temperatur der dabei entstehenden Mischung konstant zwischen 60 und 80 °C gehalten wird und wobei die in der ersten Kristallisationsstufe erhaltene 60 bis 80 °C warme D,L-Methionin und D,L-Methioninammoniumsalz enthaltende Suspension in einer zweiten Kristallisationsstufe in eine 30 bis 50 °C warme D,L-Methionin und DL-Methioninammoniumsalz enthaltende Suspension eingeleitet wird, wobei die Temperatur der dabei entstehenden Mischung konstant zwischen 30 und 50 °C gehalten wird. Dabei kann der Anteil an Verunreinigungen in der Kristallisationsbrühe in besonders effektiver Weise kontrolliert bzw. durch Ausschleusung an geeigneter Stelle abgereichert werden, ohne dass der vorteilhafte Effekt des Schüttgewichtes über 550 g/l aufgegeben wird.

Eine außerdem bevorzugte Ausführungsform des erfindungsgemäßen Verfahrens ist dadurch gekennzeichnet, dass die Kristallisation durch Vakuumkristallisation erfolgt, wobei der Druck in der ersten Kristallisationsstufe 60 bis 1000 mbar und, falls eine zweistufige Kristallisation durchgeführt wird, in der zweiten Kristallisationsstufe 35 bis 200 mbar beträgt. Dies hat den Vorteil, dass weniger kalte Oberflächen eingesetzt werden. Kalte Oberflächen können zu unerwünschten lokalen Anbackungen führen.

Die für das erfindungsgemäße Kristallisationsverfahren verwendeten D,L-Methionin- und D,L-Methioninammoniumsalze enthaltenden wässrigen Lösungen und/oder Suspensionen können zuvor durch Auflösen und/oder Suspendieren von D,L-Methionin in Wasser in Gegenwart von entsprechenden Mengen Ammoniak hergestellt werden. Das

Methionin kann aus einem beliebigen Herstellungsprozess stammen, was das Verfahren universell einsetzbar macht.

Als vorteilhaft dabei hat sich erwiesen, dass zum Auflösen D,L-Methionin mit einem Methioningehalt von mindestens 90 Gew.-%, vorzugsweise mindestens 95 Gew.-%, verwendet wird.

Geeignet dafür ist insbesondere D,L-Methionin in Form von Reinmethionin und/oder Rohmethionin aus einem beliebigen Herstellungsprozess mit einer Restfeuchte von 0,1 bis 9,5 Gew.-%, vorzugsweise 0,2 bis 4,5 Gew.-% . Insbesondere die Verwendung von noch filterfeuchten Rein- bzw. Roh-Methioninqualitäten aus einem technischen Prozess zur Herstellung von D,L-Methionin ist vorteilhaft, weil so direkt D,L-Methionin mit den gewünschten Eigenschaften insbesondere dem Schüttgewicht > 550 kg/l am Ende des Prozesses nach Trocknung erhalten werden kann.

Das erfindungsgemäße Verfahren eignet sich auch besonders für die Isolierung von D,L-Methionin aus D,L-Methionin und D,L-Methioninammoniumsalze enthaltenden wässrigen Lösungen und/oder Suspensionen, welche durch Hydrolyse von D,L-Methioninnitril und/oder D,L-Methioninamid hergestellt worden sind und zwar ohne Verwendung von salzbildenden sauren oder basischen Verseifungsagentien (wie zum Beispiel HCl, $H_2SO_4$ oder Alkalihydroxide wie NaOH oder KOH) mit Ausnahme von Ammoniak. Solche nichtsalzbildende Verseifungsagentien bzw. Verseifungskatalysatoren wie z.B.$TiO_2$ oder $MnO_2$ sind aus der einschlägigen Patentliteratur bekannt und führen ebenso wie Ammoniak als Verseifungsagens in der Verseifung von D,L-Methioninnitril und/oder D,L-Methioninamid direkt zu D,L-Methionin und D,L-Methioninammoniumsalze enthaltenden wässrigen Lösungen und/oder Suspensionen, aus denen D,L-Methionin nach dem erfindungsgemäßen Verfahren durch Kristallisation gewonnen werden kann. Solche wässrigen Lösungen und/oder Suspension enthalten daher praktisch keine Alkali-Ionen, wie zum Beispiel $Na^+$-bzw. $K^+$-Ionen.

Demgemäß ist ein weiterer Gegenstand der Erfindung ein

Verfahren zur Herstellung von D,L-Methionin, bei dem zunächst durch Hydrolyse von D,L-Methioninnitril und/oder D,L-Methioninamid eine wässrige Lösung von D,L-Methioninammoniumsalz gebildet wird und daraus durch teilweise Entfernung des Ammoniaks aus dem D,L-Methioninammoniumsalz, welcher darin gebunden als Ammonium-Ion vorliegt, eine D,L-Methionin und D,L-Methioninammoniumsalz enthaltende wässrige Lösung und/oder Suspension erzeugt wird, aus der dann D,L-Methionin durch das erfindungsgemäße Kristallisationsverfahren gewonnen wird.

Bei dem erfindungsgemäßen Verfahren wird die primär gebildete Ammoniummethioninatlösung also zunächst einer Ammoniakabreicherung unterzogen. Verfahren hierzu sind bekannt: Dies kann zum Beispiel über Erhitzen bei vermindertem Druck oder über Strippen mit Dampf erreicht werden.

Demgemäß wird das erfindungsgemäße Verfahren zur Herstellung von D,L-Methionin bevorzugt so durchgeführt, dass die wässrige Lösung von D,L-Methioninammoniumsalz vor der Kristallisation durch Eindampfen und/oder durch Abstrippen von Ammoniak auf einen $NH_4^+$-Gehalt von 1 - 5 g/kg der Lösung und/oder Suspension, vorzugsweise von 1,5 bis 3,0 g/kg, gebracht wird, z.B. über Erhitzen bei vermindertem Druck oder über Strippen mit Dampf.

Dabei wird erfindungsgemäß die Ammoniumkonzentration aber nur auf Werte in einem Bereich von ca. 5 bis ca. 60 g $NH_3$/kg Methionin abgereichert. Dabei ist es vorteilhaft die Temperatur so hoch zu wählen, dass während des Entfernens von Ammoniak nicht bereits D,L-Methionin ausfällt, sondern in Lösung verbleibt. Die heiße Methioninlösung wird vorzugsweise durch Einleiten in eine vorgelegte, kühlere Methioninsuspension rasch abgekühlt, wodurch eine Überkonzentration von gelöstem D,L-Methionin erzeugt wird und D,L-Methionin aus der Lösung ausfällt.

Dabei kann auch eine Verfahrensweise angewendet werden, bei der die wässrige Lösung und/oder Suspension von D,L-Methionin und/oder D,L-Methioninammoniumsalz durch Zugabe von Wasser und/oder D,L-Methionin auf den geeigneten Met-Gehalt von 70 - 180 g/kg der Lösung, vorzugsweise von 90 bis 150 g/kg der Lösung, gebracht wird, was das Verfahren variabel und flexibel verwendbar macht.

Das ausgefallene D,L-Methionin wird bevorzugt von der dabei entstandenen Mutterlauge getrennt und getrocknet oder zunächst umkristallisiert und nach Abtrennung von der dabei entstehenden Mutterlauge getrocknet, wobei schließlich D,L-Methionin mit einer Reinheit von mindestens 99 Gew. % und einem Schüttgewicht von mindestens 550 g/l nach Trocknung erhalten wird.

Die dabei erhaltene Mutterlauge wird vorzugsweise in eine Kristallisationsstufe zurückgeführt, was zur Minimierung von Verlusten an Methionin führt.

Das Verfahren kann sowohl kontinuierlich als auch diskontinuierlich (im batch) durchgeführt werden.

Figur 1 zeigt beispielhaft und schematisch die kontinuierliche Durchführung des erfindungsgemäßen Verfahrens. Zunächst wird die Ammoniummethioninatlösung in eine geeignete Apparatur A zur Reduktion der Ammoniakkonzentration geleitet. Dabei handelt es sich in der Regel um ein Eindampfsystem, z. B. enthaltend einen Fallfilm- oder Umlaufverdampfer. Hier werden die Bedingungen so gewählt, dass im Produktstrom eine $NH_3$ Menge von 1 bis 5 g/kg der Lösung und/oder Suspension enthalten ist, während die Met-Konzentration in einem Bereich von 70 - 180 g/kg der Lösung und/oder Suspension, bevorzugt 90 - 150 g/kg liegt. Diesem Produktstrom wird kontinuierlich das erfindungsgemäße Kristallisationsadditiv ggf. inklusive Entschäumer zugesetzt. Die Temperatur der D,L-Methionin und/oder D,L-Methioninammoniumsalz enthaltenden Lösung und/oder Suspension beträgt bevorzugt 90 bis 100 °C. Diese Met-Lösung und/oder Suspension kann bei Bedarf über einen oder mehrere Wärmetauscher B auf 100 bis 110°C erwärmt und anschließend in einer geeigneten Kristallisationsapparatur C vorzugsweise schnell, ein- oder mehrstufig auf Temperaturen zwischen 30 und 50°C abgekühlt werden, wobei das D,L-Methionin auskristallisiert. Bei Bedarf kann die D,L-Methioninsuspension in einen Zwischenbehälter D geleitet werden, um ein Nachfällen von D,L-Methionin zu ermöglichen. Das D,L-Methionin wird schließlich in einem geeigneten Fest/Flüssigtrennschritt E, wie z. B. einer Filtration oder Zentrifugation, isoliert, wobei das dabei gewonnene Filtrat bei Bedarf in den Zulauf zu Apparatur A zurückgeführt werden kann. Dabei kann es zu einer Anreicherung der erfindungsgemäßen Additive kommen.

Die nachfolgenden Beispiele sollen die Erfindung näher erläutern ohne diese jedoch zu beschränken.

**Beispiele:**

Beispiel 1: Additivscreening anionische Tenside

In einem Kolben wurden 40 g D,L-Methionin und 360 g Wasser vorgelegt und bei einer Temperatur von 40°C im Kreislauf über einen Wärmetauscher umgepumpt. Zu dieser Suspension wurde mit einer Geschwindigkeit von 18 ml/min eine auf 90 °C erhitzte Lösung aus 125 g D,L-Methionin in 1125 g Wasser gegeben, wobei die Temperatur der vorgelegten Suspension bei 40°C gehalten wurde. Nach Zugabe von 650 ml der heißen Lösung wurden 500 ml Suspension entnommen und anschließend weitere 500 ml der heißen Lösung mit einer Geschwindigkeit von 18 ml/min zudosiert. Die entstandene Suspension wurde abgelassen, die Schaummenge in ml bestimmt, das D,L-Methionin abfiltriert und mit 300 ml Aceton gewaschen. Nach Trocknung des D,L-Methionins wurde das Schüttgewicht bestimmt.

Für die Versuche mit $NH_3$ wurde die gewünschte Konzentration als $NH_4^+$-Konzentration berechnet und in beiden Startlösungen/-suspensionen eingestellt. Zusätzlich wurde die Methioninmenge äquimolar zur zugegebenen $NH_3$-Menge erhöht.

Die Kristallisationsversuche wurden in Gegenwart der folgenden Zusätze durchgeführt, wobei die angegebene Konzentration durch Zugabe des Additivs in beiden Startlösungen/-suspensionen eingestellt wurde.

Zusatz 1

Als reiner Entschäumer 1 (Zusatz 1, Vergleichsbeispiel) wurde eine wässrige Mischung enthaltend 6,9 Gewichts-% Silikonöl mit einer kinematischen Viskosität von 1000 $mm^2/s$ (AK 1000, Wacker-Chemie GmbH), 0,27 Gewichts-% hydrophobierter Kieselsäure (Sipernat D10, Evonik Degussa GmbH) und 17,9 Gewichts-% eines polyethoxylierten Fettsäuregemisches (Intrasol® FS 18/90/7, Ashland Deutschland GmbH) eingesetzt.

Als reine Kristallisationsadditive wurden die folgenden anionischen Tenside eingesetzt:

Zusatz 2) $C_nH_{2n+1}$-O-$SO_3$Na, mit n = 12 bis 18 (Sulfopon® 1218G, Oleochemicals)
Zusatz 3) $C_nH_{2n+1}$-O-$C_2H_4$-$SO_3$Na, mit n = 8 bis 18 (Hostapon® SCI 85, Clariant)
Zusatz 4) $C_nH_{2n+1}$-$(OC_2H_4)_2$-O-$SO_3$Na, mit n = 12 (Disponil® FES 27, Cognis)
Vergleichsbeispiel, Zusatz 5) $C_nH_{2n+1}$-$(OC_2H_4)_{12}$-O-$SO_3$Na, mit n = 12 (Disponil® FES 993, Cognis)
Vergleichsbeispiel, Zusatz 6) $C_nH_{2n+1}$-$(OC_2H_4)_{30}$-O-$SO_3$Na, mit n = 12 (Disponil® FES 77, Cognis)

Für die Kombination der Kristallisationsadditive mit einem Entschäumer wurde eine wässrige Mischung 7 enthaltend 6,1 Gewichts-% Silikonöl mit einer kinematischen Viskosität von 1000 $mm^2/s$ (AK 1000, Wacker-Chemie GmbH), 0,25 Gewichts-% hydrophobierter Kieselsäure (Sipernat D10, Evonik Degussa GmbH), 2,6 Gewichts-% eines polyethoxylierten Fettsäuregemisches (Intrasol® FS 18/90/7, Ashland Deutschland GmbH), 3,7 Gewichts-% eines polyethoxylierten Fettalkoholgemisches (2,35 Gewichts-% Marlipal®, Sasol Germany GmbH, 1,35 Gewichts-% Brij C2, Croda Chemicals Europe) in Wasser eingesetzt (entspricht 12,65 Gewichts-% Wirkstoff). Diese Mischung wurde mit jeweils 5,1 Gewichts-% des entsprechenden Kristallisationsadditivs (2, 3 bzw. 4) in Wasser (entspricht 17,75 Gewichts-% Gesamtwirkstoff, zusammen mit Wasser 100 Gewichts-%) eingesetzt. Folgende Zusätze wurden eingesetzt:

$$\text{Zusatz 8)} = (7) + (2)$$

$$\text{Zusatz 9)} = (7) + (3)$$

$$\text{Zusatz 10)} = (7) + (4)$$

Das Verhältnis von Entschäumer 7: Kristallisationsadditiv (2, 3, 4) betrug jeweils 2,5:1 (auf Basis des Wirkstoffs). Die Konzentrationsangabe in Tabelle 1 gibt den Gesamtwirkstoffgehalt des Zusatzes ohne Wasser bezogen auf die

Gesamtmasse der Lösung bzw. Suspension an.

Tabelle 1:

| Zusatz | Zusatz-Konzentration (ppm)1) | $NH_4^+$-Konzentration (g/kg)1) | Schaummenge (ml) | D,L-Methionin-Schüttgewicht (g/l) |
|---|---|---|---|---|
| Kein | - | - | 550 | 453 |
| 1 (Vergleichsbeispiel) | 800 | 1 | 20-30 | 454 |
| 2 | 800 | 2,5 | 85 | 564 |
| 3 | 800 | 2,5 | 400 | 573 |
| 4 | 800 | 2,5 | 250 | 588 |
| 5 (Vergleichsbeispiel) | 800 | 2,5 | 800 | 492 |
| 6 (Vergleichsbeispiel) | 800 | 2,5 | 1000 | 463 |
| 8 | 800 | 2,5 | 0 | 630 |
| 9 | 800 | 2,5 | 0 | 634 |
| 10 | 800 | 2,5 | 20-30 | 630 |
| in der Lösung und/oder Suspension (analog Beispiel 1) | | | | |

Beispiel 2: Einfluss der $NH_4^+$-Konzentration auf das Schüttgewicht von Methionin

In einem Kolben wurden 40 g Methionin und 360 g Wasser vorgelegt und bei einer Temperatur von 40°C im Kreislauf über einen Wärmetauscher umgepumpt. Zu dieser Suspension wurde mit einer Geschwindigkeit von 18 ml/min eine auf 90 °C erhitzte Lösung aus 125 g Methionin in 1125 g Wasser gegeben, wobei die Temperatur der vorgelegten Suspension bei 40°C gehalten wurde. Nach Zugabe von 650 ml der heißen Lösung wurden 500 ml Suspension entnommen und anschließend weitere 500 ml der heißen Lösung mit einer Geschwindigkeit von 18 ml/min zudosiert. Die entstandene Suspension wurde abgelassen, die Schaummenge bestimmt, das Methionin abfiltriert und mit 300 ml Aceton gewaschen. Nach Trocknung des Methionins wurde das Schüttgewicht bestimmt.

Für die Versuche mit $NH_3$ wurde die gewünschte Konzentration als $NH_4^+$-Konzentration berechnet und in beiden Startlösungen/-suspensionen durch Zugabe von wässriger $NH_3$-Lösung eingestellt.

Die Kristallisationsversuche wurden in Gegenwart des Zusatzes 8 (gemäß Beispiel 1) durchgeführt, wobei die angegebene Konzentration durch Zugabe des Zusatzes ebenfalls in beiden Startlösungen/-suspensionen gemäß Tabelle 2 eingestellt wurde.

Die Konzentrationsangabe in Tabelle 2 gibt den Gesamtwirkstoffgehalt des Zusatzes ohne Wasser bezogen auf die Gesamtmasse der Lösung bzw. Suspension an.

Tabelle 2:

| Nr. | Zusatz | Zusatz-Konzentration (ppm)1) | $NH_4^+$-Konzentration (g/kg)1) | Schaummenge (ml) | D,L-Methionin-Schüttgewicht (g/l) |
|---|---|---|---|---|---|
| 1 | Kein (Vergleich) | - | - | 350 | 434 |
| 2 | 8 (Vergleich) | 800 | - | 200 | 220 |
| 3 | 8 (Vergleich) | 800 | 0,25 | 0 | 420 |

(fortgesetzt)

| Nr. | Zusatz | Zusatz-Konzentration (ppm)[1)] | $NH_4^+$-Konzentration (g/kg)[1)] | Schaummenge (ml) | D,L-Methionin-Schüttgewicht (g/l) |
|---|---|---|---|---|---|
| 4 | 8 (Vergleich) | 800 | 0,5 | 0 | 505 |
| 5 | 8 | 800 | 1 | 0 | 586 |
| 6 | 8 | 800 | 2,5 | 0 | 615 |
| [1)] in der Lösung und/oder Suspension (analog Beispiel 1) | | | | | |

Beispiel 3: Konzentrationsabhängige Experimente

In einem Kolben wurden 48 g D,L-Methionin, 348 g Wasser und 3,8 g wässrige $NH_3$-Lösung (25%ig) vorgelegt und bei einer Temperatur von 40°C im Kreislauf über einen Wärmetauscher umgepumpt. Zu dieser Suspension wurde mit einer Geschwindigkeit von 18 ml/min eine auf 90 °C erhitzte Lösung aus 151 g D,L-Methionin, 1087 g Wasser und 11,8 g wässrige $NH_3$-Lösung (25%ig) gegeben, wobei die Temperatur der vorgelegten Suspension bei 40°C gehalten wurde. Nach Zugabe von 650 ml der heißen Lösung wurden 500 ml Suspension entnommen und anschließend weitere 500 ml der heißen Lösung mit einer Geschwindigkeit von 18 ml/min zudosiert. Die entstandene Suspension wurde abgelassen, die Schaummenge bestimmt, das D,L-Methionin abfiltriert und mit 300 ml Aceton gewaschen. Nach Trocknung des D,L-Methionins wurde das Schüttgewicht bestimmt.

Die Kristallisationsversuche wurden in Gegenwart der folgenden Zusätze 8, 9 bzw. 10 (gem. Beispiel 1) durchgeführt, wobei die angegebene Konzentration durch Zugabe des Zusatzes in beiden Startlösungen/-suspensionen gemäß Tabelle 4 eingestellt wurde.

Zusatz 8) (7) + (2)

Zusatz 9) (7) + (3)

Zusatz 10) (7) + (4)

Die Konzentrationsangabe in Tabelle 3 gibt den Gesamtwirkstoffgehalt des Zusatzes ohne Wasser bezogen auf die Gesamtmasse der Lösung bzw. Suspension an.

Tabelle 3:

| Nr. | Zusatz | Zusatz-Konzentration (ppm) [1)] | Schaummenge (ml) | D,L-Methionin-Schüttgewicht (g/l) |
|---|---|---|---|---|
| 1 | 8 | 100 | 350 | 522 |
| 2 | 8 | 200 | 180 | 561 |
| 3 | 8 | 400 | 5 | 610 |
| 4 | 8 | 800 | 0 | 630 |
| 5 | 8 | 1200 | 0 | 624 |
| 6 | 8 | 2000 | 0 | 615 |
| 7 | 8 | 4000 | 0 | 616 |
| 8 | 9 | 100 | 450-500 | 529 |
| 9 | 9 | 200 | 200 | 585 |
| 10 | 9 | 400 | 20-30 | 625 |
| 11 | 9 | 800 | 0 | 634 |

(fortgesetzt)

| Nr. | Zusatz | Zusatz-Konzentration (ppm) 1) | Schaummenge (ml) | D,L-Methionin-Schüttgewicht (g/l) |
|---|---|---|---|---|
| 12 | 9 | 1200 | 0 | 657 |
| 13 | 9 | 2000 | 0 | 630 |
| 14 | 9 | 4000 | 0 | 601 |
| 15 | 10 | 100 | 350 | 507 |
| 16 | 10 | 200 | 150 | 558 |
| 17 | 10 | 400 | 80 | 614 |
| 18 | 10 | 800 | 20-30 | 630 |
| 19 | 10 | 1200 | 20-30 | 652 |
| 20 | 10 | 2000 | 5-10 | 650 |
| 21 | 10 | 4000 | 0 | 636 |
| 1)in der Lösung und/oder Suspension (analog Beispiel 1) | | | | |

Man erkennt, dass die erfindungsgemäßen Zusätze (mit Entschäumer) über den Konzentrationsbereich von 400 bis 4000 ppm das Schüttgewicht des D,L-Methionins auf Werte > 600 g/l verbessern.

## Patentansprüche

**1.** Verfahren zur Kristallisation von D,L-Methionin aus D,L-Methionin und D,L-Methioninammoniumsalz enthaltenden wässrigen Lösungen und/oder Suspensionen mit einem Met-Gehalt von 70 - 180 g/kg der Lösung und/oder Suspension, vorzugweise 90 - 150 g/kg der Lösung und/oder Suspension, einem $NH_4^+$-Gehalt von 1 - 5 g/kg der Lösung und/oder Suspension, in Gegenwart eines Kristallisationsadditivs,
welches ein anionisches Tensid oder eine Mischung aus verschiedenen anionischen Tensiden umfasst,
bei dem die Temperatur der Lösung und/oder Suspension von $T_1$ = 85 - 110°C direkt oder schrittweise auf $T_2$ = 30 - 50°C abgesenkt wird, so dass D,L-Methionin als Feststoff ausfällt, **dadurch gekennzeichnet, dass** das zur Bildung des Kristallisationsadditiv eingesetzte anionische Tensid eine der in Formel 1 bis 3 dargestellten Verbindungen, oder eine Mischung daraus ist:

$R^1$-O-$SO_3M$ (Formel 1)

$R^2$-O-$(CH_2)_n$-$SO_3M$ (Formel 2)

$R^3$-$(OC_2H_4)_n$-O-$SO_3M$ (Formel 3)

wobei n eine ganze Zahl von 1 bis 10, M Natrium oder Kalium und $R^1$, $R^2$ und $R^3$ eine lineare, verzweigte oder zyklische, gesättigte oder ungesättigte $C_8$-bis $C_{20}$-Alkylgruppe oder eine Arylgruppe ist.

**2.** Verfahren nach Anspruch 1, **dadurch gekennzeichnet, dass** das n gleich 2 und $R^1$, $R^2$ und $R^3$ lineare, gesättigte $C_8$-bis $C_{18}$-Alkylgruppen sind.

**3.** Verfahren gemäß Anspruch 1 oder 2, **dadurch gekennzeichnet, dass**
$C_nH_{2n+1}$-O-$SO_3Na$, mit n = 12 bis 18,
$C_nH_{2n+1}$-O-$C_2H_4$-$SO_3Na$, mit n = 8 bis 18 oder
$C_nH_{2n+1}$-$(OC2H_4)_2$-O-$SO_3Na$, mit n = 12
als anionisches Tensid eingesetzt werden.

**4.** Verfahren nach einem der Ansprüche 1 bis 3, **dadurch gekennzeichnet, dass** die Konzentration des Kristallisationsadditivs in der Lösung und/oder Suspension, aus der die Kristallisation erfolgt, mindestens 700 ppm und maximal 4000 ppm, bezogen auf die Gesamtmasse der Lösung und/oder Suspension beträgt.

**5.** Verfahren gemäß Anspruch 4, **dadurch gekennzeichnet dass**, die Konzentration des Kristallisationsadditivs in der Lösung und/oder Suspension vorzugsweise mindestens 750 ppm und maximal 2000 ppm, beträgt.,

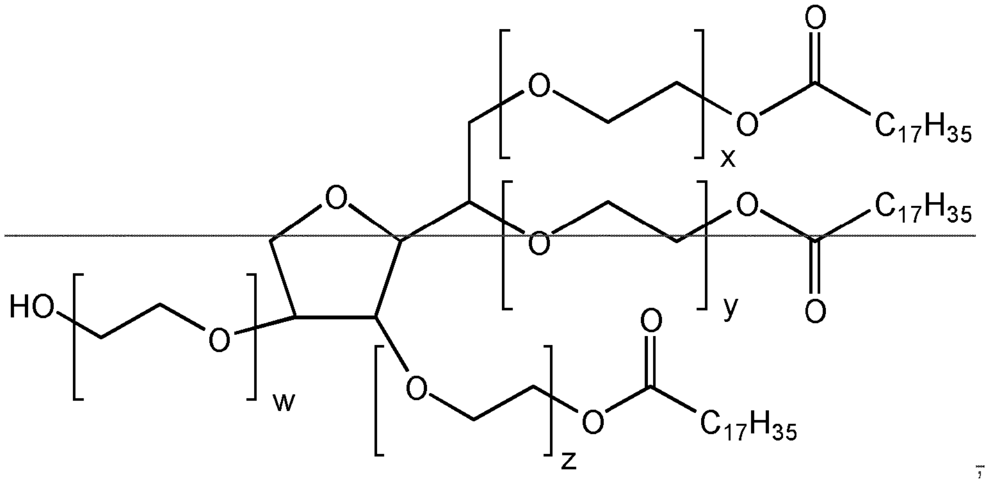

**6.** Verfahren gemäß Anspruch 5, **dadurch gekennzeichnet dass**, wie Konzentration des Kristallisationsadditivs mindestens 800 ppm und maximal 1000 ppm beträgt.

**7.** Verfahren nach einem der Ansprüche 1 bis 6, **dadurch gekennzeichnet, dass** die Lösung, aus der die Kristallisation erfolgt, zusätzlich einen Entschäumer enthält.

**8.** Verfahren nach Anspruch 7, **dadurch gekennzeichnet, dass** der Entschäumer Silikonöl enthält.

**9.** Verfahren gemäß Anspruch 7 oder 8, **dadurch gekennzeichnet, dass** das Gewichtsverhältnis Entschäumer: Kristallisationsadditiv auf Basis des Wirkstoffs im Bereich von 4:1 bis 1:1, vorzugsweise im Bereich von 3:1 bis 2:1 liegt und dabei die Konzentration des Kristallisationsadditivs mindestens 50 ppm und maximal 1200 ppm, bezogen auf die Gesamtmasse der Lösung und/oder Suspension beträgt, vorzugsweise 100 ppm bis 600 ppm, besonders bevorzugt 200 ppm bis 400 ppm.

**10.** Verfahren nach einem der Ansprüche 1 bis 9, **dadurch gekennzeichnet, dass** die Kristallisation durch Einleiten einer 85 bis 110°C heißen D,L-Methionin und D,L-Methioninammoniumsalz enthaltenden wässrigen Lösung und/oder Suspension in eine 30 bis 50°C warme D,L-Methionin und D,L-Methioninammoniumsalz enthaltende wässrige Lösung und/oder Suspension erfolgt, wobei die Temperatur der dabei entstehenden Mischung konstant zwischen 30 und 50°C gehalten wird.

**11.** Verfahren gemäß Anspruch 10, **dadurch gekennzeichnet, dass** die Kristallisation durch Einleiten einer 85 bis 110°C heißen D,L-Methionin und D,L-Methioninammoniumsalz enthaltenden Lösung in eine 30 bis 50°C warme D,L-Methionin und D,L-Methioninammoniumsalz enthaltende Suspension erfolgt.

**12.** Verfahren nach einem der Ansprüche 1 bis 9, **dadurch gekennzeichnet, dass** die Kristallisation zweistufig durchgeführt wird, wobei in der ersten Kristallisationsstufe eine 85 bis 110°C heiße D,L-Methionin und D,L-Methioninammoniumsalz enthaltende Lösung und/oder Suspension in eine 60 bis 80 °C warme D,L-Methionin und DL-Methioninammoniumsalz enthaltende Suspension eingeleitet und die Temperatur der dabei entstehenden Mischung konstant zwischen 60 und 80 °C gehalten wird und wobei die in der ersten Kristallisationsstufe erhaltene 60 bis 80 °C warme D,L-Methionin und D,L-Methioninammoniumsalz enthaltende Suspension in einer zweiten Kristallisationsstufe in eine 30 bis 50 °C warme D,L-Methionin und D,L-Methioninammoniumsalz enthaltende Suspension eingeleitet wird, wobei die Temperatur der dabei entstehenden Mischung konstant zwischen 30 und 50 °C gehalten wird.

**13.** Verfahren nach einem der Ansprüche 1 bis 12, **dadurch gekennzeichnet, dass** die Kristallisation durch Vakuumkristallisation erfolgt, wobei der Druck in der Kristallisationsstufe 60 bis 1000 mbar und, falls eine zweistufige Kristallisation durchgeführt wird, in der zweiten Kristallisationsstufe 35 bis 200 mbar beträgt.

**14.** Verfahren nach einem der Ansprüche 1 bis 13, **dadurch gekennzeichnet, dass** die D,L-Methionin- und D,L-Methioninammoniumsalze enthaltenden wässrigen Lösungen und/oder Suspensionen zuvor durch Auflösen und/oder Suspendieren von D,L-Methionin in Wasser in Gegenwart von entsprechenden Mengen Ammoniak hergestellt wurden.

**15.** Verfahren nach Anspruch 14, **dadurch gekennzeichnet, dass** zum Auflösen D,L-Methionin mit einem Methioningehalt von mindestens 90 Gew.-%, vorzugsweise mindestens 95 Gew.-%, verwendet wird.

**16.** Verfahren gemäß Anspruch 15, **dadurch gekennzeichnet, dass** D,L-Methionin in Form von Reinmethionin und/oder Rohmethionin aus einem beliebigen Herstellungsprozess mit einer Restfeuchte von 0,1 bis 9,5 Gew.-%, vorzugsweise 0,2 bis 4,5 Gew.-% verwendet wird.

**17.** Verfahren zur Herstellung von D,L-Methionin, bei dem zunächst durch Hydrolyse von D,L-Methioninnitril und/oder D,L-Methioninamid eine wässrige Lösung von D,L-Methioninammoniumsalz gebildet wird und daraus durch teilweise Entfernung des Ammoniaks aus dem D,L-Methioninammoniumsalz eine D,L-Methionin und D,L-Methioninammoniumsalz enthaltende wässrige Lösung und/oder Suspension erzeugt wird, aus der dann Methionin durch Kristallisation gemäß einem der Ansprüche 1 bis 16 gewonnen wird.

**18.** Verfahren gemäß Anspruch 17, **dadurch gekennzeichnet, dass** die wässrige Lösung von D,L-Methioninammoniumsalz vor der Kristallisation durch Eindampfen und/oder durch Abstrippen von Ammoniak auf einen $NH_4^+$-Gehalt von 1 - 5 g/kg der Lösung und/oder Suspension, vorzugsweise von 1,5 bis 3,0 g/kg, gebracht wird.

**19.** Verfahren gemäß Anspruch 17 oder 18, **dadurch gekennzeichnet, dass** die wässrige Lösung und/oder Suspension von D,L-Methionin und D,L-Methioninammoniumsalz durch Zugabe von Wasser und/oder D,L-Methionin auf einen Met-Gehalt von 70 - 180 g/kg der Lösung und/oder Suspension, vorzugsweise von 90 bis 150 g/kg, gebracht wird.

**20.** Verfahren nach einem der Ansprüche 1 bis 19, **dadurch gekennzeichnet, dass** das ausgefallene D,L-Methionin von der Mutterlauge getrennt und getrocknet oder zunächst umkristallisiert und nach Abtrennung von der dabei entstehenden Mutterlauge getrocknet wird.

**21.** Verfahren nach Anspruch 20, **dadurch gekennzeichnet, dass** die Mutterlauge in eine Kristallisationsstufe zurückgeführt wird.

## Claims

**1.** Method for crystallizing D,L-methionine from aqueous solutions and/or suspensions containing D,L-methionine and D,L-methionine ammonium salt, having a Met content of 70 - 180 g/kg of solution and/or suspension, preferably 90 - 150 g/kg of solution and/or suspension, an $NH_4^+$ content of 1 - 5 g/kg of solution and/or suspension, in the presence of a crystallization additive,
which comprises an anionic surfactant or a mixture of various anionic surfactants,
in which the temperature of the solution and/or suspension is lowered directly or stepwise from $T_1$ = 85 - 110°C to $T_2$ = 30 - 50°C, so that D,L-methionine precipitates as a solid, **characterized in that** the anionic surfactant used for the formation of the crystallization additive is one of the compounds depicted in formulae 1 to 3 or a mixture thereof:

$R^1$-O-$SO_3M$ (formula 1)

$R^2$-O- $(CH_2)_n$-$SO_3M$ (formula 2)

$R^3$-$(OC_2H_4)_n$-O-$SO_3M$ (formula 3)

wherein n is an integer from 1 to 10, M is sodium or potassium and $R^1$, $R^2$ and $R^3$ are a linear, branched or cyclic, saturated or unsaturated $C_8$- to $C_{20}$-alkyl group or an aryl group.

**2.** Method according to Claim 1, **characterized in that** n is equal to 2 and $R^1$, $R^2$ and $R^3$ are linear, saturated $C_8$- to $C_{18}$-alkyl groups.

**3.** Method according to Claim 1 or 2, **characterized in that**
$C_nH_{2n+1}$-O-$SO_3Na$, where n = 12 to 18,

$C_nH_{2n+1}$-O-$C_2H_4$-$SO_3Na$, where n = 8 to 18 or
$C_nH_{2n+1}$-$(OC_2H_4)_2$-O-$SO_3Na$, where n = 12 are used as anionic surfactant.

**4.** Method according to any of Claims 1 to 3, **characterized in that** the concentration of the crystallization additive in the solution and/or suspension from which the crystallization takes place is at least 700 ppm and not more than 4000 ppm, based on the total mass of the solution and/or suspension.

**5.** Method according to Claim 4, **characterized in that** the concentration of the crystallization additive in the solution and/or suspension is preferably at least 750 ppm and not more than 2000 ppm.

**6.** Method according to Claim 5, **characterized in that** the concentration of the crystalline additive is least 800 ppm and not more than 1000 ppm.

**7.** Method according to any of Claims 1 to 6, **characterized in that** the solution from which the crystallization takes place additionally comprises a defoamer.

**8.** Method according to Claim 7, **characterized in that** the defoamer comprises silicone oil.

**9.** Method according to Claim 7 or 8, **characterized in that** the weight ratio of defoamer: crystallization additive, based on the active ingredient, is in the range of 4:1 to 1:1, preferably in the range of 3:1 to 2:1 and in this case the concentration of the crystallization additive is at least 50 ppm and not more than 1200 ppm, based on the total mass of the solution and/or suspension, preferably 100 ppm to 600 ppm, particularly preferably 200 ppm to 400 ppm.

**10.** Method according to any of Claims 1 to 9, **characterized in that** the crystallization takes place by introducing an aqueous solution and/or suspension containing D,L-methionine and D,L-methionine ammonium salt heated to 85 to 110°C into an aqueous solution and/or suspension containing D,L-methionine and D,L-methionine ammonium salt warmed to 30 to 50°C, wherein the temperature of the resulting mixture is constantly maintained between 30 and 50°C.

**11.** Method according to Claim 10, **characterized in that** the crystallization takes place by introducing a solution containing D,L-methionine and D,L-methionine ammonium salt heated to 85 to 110°C into a suspension containing D,L-methionine and D,L-methionine ammonium salt warmed to 30 to 50°C.

**12.** Method according to any of Claims 1 to 9, **characterized in that** the crystallization is carried out in two stages, wherein, in the first crystallization stage, a solution and/or suspension containing D,L-methionine and D,L-methionine ammonium salt heated to 85 to 110°C is introduced into a suspension containing D,L-methionine and D,L-methionine ammonium salt warmed to 60 to 80°C and the temperature of the resulting mixture is constantly maintained between 60 and 80°C and wherein the suspension containing D,L-methionine and D,L-methionine ammonium salt warmed to 60 to 80°C obtained in the first crystallization stage is introduced in a second crystallization stage into a suspension containing D,L-methionine and D,L-methionine ammonium salt warmed to 30 to 50°C, wherein the temperature of the resulting mixture is constantly maintained between 30 and 50°C.

**13.** Method according to any of Claims 1 to 12, **characterized in that** the crystallization is carried out by vacuum crystallization, wherein the pressure in the crystallization stage is 60 to 1000 mbar and, if a two-stage crystallization is carried out, the pressure in the second crystallization stage is 35 to 200 mbar.

**14.** Method according to any of Claims 1 to 13, **characterized in that** the aqueous solutions and/or suspensions containing D,L-methionine and D,L-methionine ammonium salts were prepared beforehand by dissolving and/or suspending D,L-methionine in water in the presence of appropriate amounts of ammonia.

**15.** Method according to Claim 14, **characterized in that** D,L-methionine having a methionine content of at least 90% by weight, preferably at least 95% by weight, is used for the dissolution.

**16.** Method according to Claim 15, **characterized in that** D,L-methionine is used in the form of pure methionine and/or crude methionine from any manufacturing process having a residual moisture of 0.1 to 9.5% by weight, preferably 0.2 to 4.5% by weight.

**17.** Method for preparing D,L-methionine, in which an aqueous solution of D,L-methionine ammonium salt is initially

formed by hydrolysis of D,L-methionine nitrile and/or D,L-methionine amide, and an aqueous solution and/or suspension containing D,L-methionine and D,L-methionine ammonium salt is produced therefrom by partial removal of the ammonia from the D,L-methionine ammonium salt, from which methionine is then produced by crystallization according to any of Claims 1 to 16.

**18.** Method according to Claim 17, **characterized in that** the aqueous solution of D,L-methionine ammonium salt is brought to an $NH_4^+$ content of 1 - 5 g/kg of solution and/or suspension, preferably 1.5 to 3.0 g/kg, by evaporating and/or stripping off of ammonia prior to the crystallization.

**19.** Method according to Claim 17 or 18, **characterized in that** the aqueous solution and/or suspension of D,L-methionine and D,L-methionine ammonium salt is brought to a Met content of 70 - 180 g/kg of solution and/or suspension, preferably 90 to 150 g/kg, by addition of water and/or D,L-methionine.

**20.** Method according to any of Claims 1 to 19, **characterized in that** the precipitated D,L-methionine is separated from the mother liquor and dried or is initially recrystallized and is dried after separation from the mother liquor obtained in this case.

**21.** Method according to Claim 20, **characterized in that** the mother liquor is fed back into a crystallization stage.

## Revendications

**1.** Procédé pour la cristallisation de D,L-méthionine à partir de solutions et/ou de suspensions aqueuses contenant de la D,L-méthionine et du sel d'ammonium de D,L-méthionine dotées d'une teneur en Met de 70 à 180 g/kg de la solution et/ou de la suspension, de préférence de 90 à 150 g/kg de la solution et/ou de la suspension, d'une teneur en $NH_4^+$ de 1 à 5 g/kg de la solution et/ou de la suspension, en présence d'un additif de cristallisation, qui comprend un tensioactif anionique ou un mélange de différents tensioactifs anioniques, dans lequel la température de la solution et/ou de la suspension est abaissée de $T_1$ = 85 à 110 °C, directement ou par étapes, à $T_2$ = 30 à 50 °C, de sorte que de la D,L-méthionine précipite en tant que solide, **caractérisé en ce que** le tensioactif anionique utilisé pour la formation de l'additif de cristallisation est l'un des composés représentés dans les formules 1 à 3, ou est un mélange de ceux-ci :

$R^1$-O-$SO_3M$ (formule 1)

$R^2$-O-$(CH_2)_n$-$SO_3M$ (formule 2)

$R^3$-$(OC_2H_4)_n$-O-$SO_3M$ (formule 3)

n étant un nombre entier de 1 à 10, M étant sodium ou potassium et $R^1$, $R^2$ et $R^3$ étant un groupe $C_{8-20}$-alkyle linéaire, ramifié ou cyclique, saturé ou insaturé ou un groupe aryle.

**2.** Procédé selon la revendication 1, **caractérisé en ce que** n est égal à 2 et $R^1$, $R^2$ et $R^3$ sont des groupes $C_{8-18}$-alkyle linéaires, saturés.

**3.** Procédé selon la revendication 1 ou 2, **caractérisé en ce que**
$C_nH_{2n+1}$-O-$SO_3Na$, avec n = 12 à 18,
$C_nH_{2n+1}$-O-$C_2H_4$-$SO_3Na$, avec n = 8 à 18 ou
$C_nH_{2n+1}$-$(OC_2H_4)_2$-O-$SO_3M$, avec n = 12 sont utilisés en tant que tensioactif anionique.

**4.** Procédé selon l'une quelconque des revendications 1 à 3, **caractérisé en ce que** la concentration de l'additif de cristallisation dans la solution et/ou dans la suspension, à partir de laquelle la cristallisation est réalisée, est d'au moins 700 ppm et de maximum 4 000 ppm, par rapport à la masse totale de la solution et/ou de la suspension.

**5.** Procédé selon la revendication 4, **caractérisé en ce que** la concentration de l'additif de cristallisation dans la solution et/ou dans la suspension est de préférence d'au moins 750 ppm et de maximum 2 000 ppm.

**6.** Procédé selon la revendication 5, **caractérisé en ce que** la concentration de l'additif de cristallisation est d'au moins 800 ppm et de maximum 1 000 ppm.

7. Procédé selon l'une quelconque des revendications 1 à 6, **caractérisé en ce que** la solution, à partir de laquelle la cristallisation est réalisée, contient de plus un agent antimousse.

8. Procédé selon la revendication 7, **caractérisé en ce que** l'antimousse contient une huile de silicone.

9. Procédé selon la revendication 7 ou 8, **caractérisé en ce que** le rapport en poids antimousse : additif de cristallisation, sur la base du principe actif, se situe dans la plage de 4 : 1 à 1 : 1, de préférence dans la plage de 3 : 1 à 2 : 1 et à cet égard la concentration de l'additif de cristallisation est d'au moins 50 ppm et de maximum 1 200 ppm, par rapport à la masse totale de la solution et/ou de la suspension, de préférence de 100 ppm à 600 ppm, particulièrement préférablement de 200 ppm à 400 ppm.

10. Procédé selon l'une quelconque des revendications 1 à 9, **caractérisé en ce que** la cristallisation est réalisée par l'introduction d'une solution et/ou d'une suspension aqueuses chauffées à une température de 85 à 110 °C contenant de la D,L-méthionine et du sel d'ammonium de D,L-méthionine dans une solution et/ou une suspension aqueuses chauffées à une température de 30 à 50 °C contenant de la D,L-méthionine et du sel d'ammonium de D,L-méthionine, la température du mélange ainsi produit étant maintenue constante entre 30 et 50 °C.

11. Procédé selon la revendication 10, **caractérisé en ce que** la cristallisation est réalisée par l'introduction d'une solution chauffée à une température de 85 à 110 °C contenant de la D,L-méthionine et du sel d'ammonium de D,L-méthionine dans une suspension chauffée à une température de 30 à 50 °C contenant de la D,L-méthionine et du sel d'ammonium de D,L-méthionine.

12. Procédé selon l'une quelconque des revendications 1 à 9, **caractérisé en ce que** la cristallisation est mise en œuvre en deux étapes, dans lequel dans la première étape de cristallisation, une solution et/ou une suspension chauffées à une température de 85 à 110 °C contenant de la D,L-méthionine et du sel d'ammonium de D,L-méthionine sont introduites dans une suspension chauffée à une température de 60 à 80 °C contenant de la D,L-méthionine et du sel d'ammonium de D,L-méthionine et la température du mélange ainsi produit est maintenue constante entre 60 et 80 °C et la suspension chauffée à une température de 60 à 80 °C contenant de la D,L-méthionine et du sel d'ammonium de D,L-méthionine obtenue dans la première étape de cristallisation est introduite, dans une deuxième étape de cristallisation, dans une suspension chauffée à une température de 30 à 50 °C contenant de la D,L-méthionine et du sel d'ammonium de D,L-méthionine, la température du mélange ainsi obtenu étant maintenue constante entre 30 et 50 °C.

13. Procédé selon l'une quelconque des revendications 1 à 12, **caractérisé en ce que** la cristallisation est réalisée par cristallisation sous vide, la pression dans l'étape de cristallisation étant de 60 à 1 000 mbars et, dans le cas où une cristallisation en deux étapes est mise en œuvre, la pression dans la deuxième étape de cristallisation étant de 35 à 200 mbars.

14. Procédé selon l'une quelconque des revendications 1 à 13, **caractérisé en ce que** les solutions et/ou les suspensions aqueuses contenant de la D,L-méthionine et du sel d'ammonium de D,L-méthionine ont été préparées au préalable par dissolution et/ou mise en suspension de D,L-méthionine dans de l'eau en présence de quantités correspondantes d'ammoniaque.

15. Procédé selon la revendication 14, **caractérisé en ce que** pour la dissolution, de la D,L-méthionine dotée d'une teneur en méthionine d'au moins 90 % en poids, de préférence d'au moins 95 % en poids, est utilisée.

16. Procédé selon la revendication 15, **caractérisé en ce que** de la D,L-méthionine sous forme de méthionine pure et/ou de méthionine brute provenant d'un quelconque procédé de production dotée d'une humidité résiduelle de 0,1 à 9,5 % en poids, de préférence de 0,2 à 4,5 % en poids, est utilisée.

17. Procédé pour la préparation de D,L-méthionine, dans lequel tout d'abord une solution aqueuse du sel d'ammonium de D,L-méthionine est formée par hydrolyse de nitrile de D,L-méthionine et/ou de D,L-méthioninamide et à partir de celle-ci, une solution et/ou une suspension aqueuses contenant de la D,L-méthionine et du sel d'ammonium de D,L-méthionine est produite en éliminant partiellement l'ammoniaque du sel d'ammonium de D,L-méthionine, puis à partir de celle-ci, de la méthionine est obtenue par cristallisation selon l'une quelconque des revendications 1 à 16.

18. Procédé selon la revendication 17, **caractérisé en ce que** la solution aqueuse du sel d'ammonium de D,L-méthionine, avant la cristallisation, est amenée par évaporation et/ou par strippage d'ammoniac, à une teneur en $NH_4^+$ de 1 à

5 g/kg de la solution et/ou de la suspension, de préférence de 1,5 à 3,0 g/kg.

**19.** Procédé selon la revendication 17 ou 18, **caractérisé en ce que** la solution et/ou la suspension aqueuses de D,L-méthionine et de sel d'ammonium de D,L-méthionine sont amenées, par ajout d'eau et/ou de D,L-méthionine, à une teneur en Met de 70 à 180 g/kg de la solution et/ou de la suspension, de préférence de 90 à 150 g/kg.

**20.** Procédé selon l'une quelconque des revendications 1 à 19, **caractérisé en ce que** la D,L-méthionine précipitée est séparée des eaux mères et séchée ou d'abord recristallisée et séchée après séparation des eaux mères ainsi produites.

**21.** Procédé selon la revendication 20, **caractérisé en ce que** les eaux mères sont recyclées dans une étape de cristallisation.

**Figur 1**

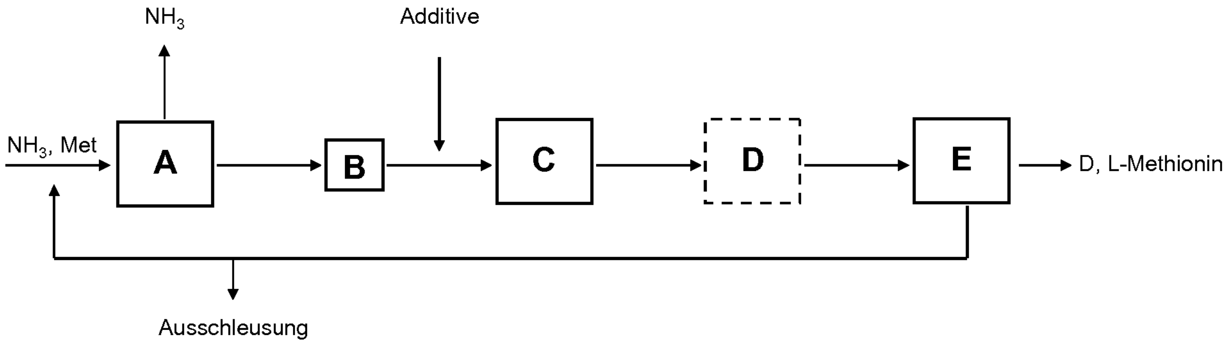

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20050176115 A
- JP 2004254690 A
- DE 60127538
- WO 2008006977 A
- WO 2007034065 A
- DE 10238212
- WO 2003050071 A